# EUROPEAN PATENT APPLICATION

(11) **EP 4 345 837 A1**
(43) Date of publication of application: **03.04.2024**
(21) Application number: 22199005.4
(22) Date of filing: 30.09.2022
(51) Int. Cl.: G16H 30/40, G16H 50/20

(54) **DETECTING THE PRESENCE OF PATHOLOGIES DURING A MEDICAL SCAN**

(71) Applicant: Koninklijke Philips N.V., 5656 AG Eindhoven (NL)
(72) Inventor: PEZZOTTI, Nicola, Eindhoven (NL); GESSERT, Nils Thorben, Eindhoven (NL)
(74) Representative: Philips Intellectual Property & Standards

(57) **Abstract**

A method for obtaining an overall score indicative of the presence of a target pathology of a subject during a medical scan procedure. The method comprises acquiring images during the medical scan procedure and iteratively identifying whether a newly acquired image corresponds to one of a set of pre-determined views. For the newly acquired images which correspond to one of the pre-determined views, a predictive model is selected from a set of predictive models each trained on one or more of the pre-determined views for the target pathology. The selected predictive model is trained on the pre-determined view or multiple predetermined views that have been acquired. The newly acquired image is input into the selected predictive model and an overall score is updated, or generated using the output of the selected predictive model. The overall score is indicative of the presence of the target pathology in the subject.

## Description

### FIELD OF THE INVENTION

The invention relates to the field of detecting pathologies during medical scans. In particular, the invention relates to the field of obtaining a score indicative of a target pathology using predictive models.

### BACKGROUND OF THE INVENTION

During a cardiac echo exam, the sonographer must acquire multiple views of the patient's heart. This can be during a transthoracic echocardiogram (TTE), transesophageal echocardiogram (TEE) or stress exam, and both in rest and stress stages. The goal is to discover potential issues in organs, such as wall motion abnormalities (WMA). After the sonographer acquires the images, the discovery of abnormalities is performed by a cardiologist by observing the different cardiac cycles and detecting abnormalities in the heart function.

It is a key focus of the field to improve the standardization of diagnoses while reducing the number of exams that cannot be analyzed by the cardiologists due to missing views and/or substandard quality.

Predictive models can be used to detect abnormalities given a complete exam (i.e., when all views are acquired and shared with the cardiologist). The advantage of using predictive models is that it will support the cardiologist in making a diagnosis, greatly increasing the standardization of reporting which is an issue in the field.

However, for such a model to function properly, all views need to be acquired before predicting abnormalities. If the sonographer forgets a view, or if the quality of a view is not sufficient, the predictive WMA model will fail. This means that a conclusive diagnosis cannot be made, and the patient needs to be re-scanned.

Moreover, during a stress examination, the patient is scanned during rest and peak phases. In the case where the patient has a WMA, the stress examination can be dangerous, and the sonographer must stop the exam. To this end, the sonographer verifies with the patient if they feel pain that they cannot endure during the exam. When the patient is overly uncomfortable, the exam is stopped.

Thus, there is a need to improve the use of predictive models during medical scans to reduce the number of re-scans and the time of required for medical scans.

### SUMMARY OF THE INVENTION

The invention is defined by the claims.

According to examples in accordance with an aspect of the invention, there is provided a method for obtaining an overall score indicative of the presence of a target pathology of a subject during a medical scan procedure, the method comprising:
acquiring images during the medical scan procedure; and
iteratively:
   identifying whether a newly acquired image corresponds to one of a set of pre-determined views;
   for a newly acquired image which does correspond to one of the pre-determined views:
      selecting a predictive model trained on the pre-determined view or multiple predetermined views that have been acquired, from a set of predictive models each trained on one or more of the pre-determined views for the target pathology;
      inputting the newly acquired image into the selected predictive model; and
      generating or updating an overall score indicative of the presence of the target pathology using the output of the selected predictive model.

The first overall score may be the first output from the predictive models which is obtained. Alternatively, the overall score may start at, for example, 0 and be updated each time a new image is assessed.

The medical scan procedure may be any medical scan which acquires images of the subject over time.

Iteratively updating the overall score during the medical scan provides the clinician performing the medical scan with real time updates on whether the target pathology is present. This can prevent situations where the medical scan needs to be repeated if not enough images, at the pre-determined views, were acquired.

Additionally, the overall score being dynamically updated with the acquisition of the images enables the clinician to stop the scan if they believe it may be causing harm to the subject. For example, cardiac scans often involve stress exams where the subject is told to perform exercise to increase their heart rate. If the subject has a heart abnormality, the stress exam may be causing harm to the subject. As such, iteratively updating the overall score can enable the clinician to stop the stress exam if they believe it may cause harm to the subject (e.g. because the overall score indicates the target heart pathology is present).

Note that the overall score does not need to be a numeric value. It may be a general indication such as "absent", "mild", "severe", "extremely severe" or indeed any suitable way to convey information about a target pathology. For example, color coding may be used to indicate level of severity or likelihood of a pathology being present.

The method may further comprise providing an alert to a clinician performing the medical scan procedure in response to the overall score exceeding a pre-determined threshold and/or the number of acquired images, corresponding to distinct pre-determined views, exceeding a pre-determined view threshold.

The overall score exceeding a pre-determined threshold could indicate that the target pathology is highly likely to be present. This enables the clinician to decide to end the scan sooner than expected. As mentioned above, ending the scan sooner may prevent harm from being done to the subject.

Similarly, once a pre-determined number of different views has been obtained, the scan can usually be stopped. Often, the pre-determined number of views is the maximum number of views which can be analyzed by the predictive models and thus there would a minimal effect to the overall score by continuing the scan. As such, providing an alert to the clinician when the pre-determined number of views has been reached can enable the clinician to decide to end the scan sooner than planned.

The medial scan, and hence the method of obtaining a score, may be automatically stopped when the number of views exceeds the pre-determined view threshold.

The method may further comprise iteratively updating an overall confidence measure, indicating the confidence in the overall score.

The method may further comprise inputting the newly acquired image into the selected predictive model to obtain a per-image score indicative of the presence of a target pathology in the newly acquired image and a per-image confidence measure for the corresponding per-image score and generating or updating the overall score and the overall confidence measure using the per-image score and per-image confidence measure.

Providing an overall confidence measure enables the clinician to be more (or less) confident in the overall score and thus decide whether the scan should be continued (e.g. if the overall confidence measure is relatively low) or if it can be ended (e.g. if the overall confidence measure is relatively high).

Iteratively updating the overall score may comprise iteratively averaging the latest available per-image score with the overall score.

Averaging may comprise determining the arithmetic, geometric or harmonic mean of the latest available per-image score and the overall score. Other averaging techniques could also be used.

The method may comprise, in response to two or more of the acquired images corresponding to distinct pre-determined views, selecting a predictive model trained on the two or more corresponding pre-determined views from the set of predictive models and inputting the two or more acquired images into the selected predictive model to assess the presence of the target pathology in the two or more acquired images.

Often, predictive models can use multiple distinct views when predicting the presence of a target pathology. It has been found that these predictive models provide more robust predictions than predictive models which use a single view.

In some cases, it may be beneficial to use a combination of single-view predictive models (as they require fewer views and can thus provide per-image scores more quickly) and multiple-view predictive models (as they provide more robust per-image scores but take longer to provide the per-image scores).

The medical scan procedure may be a cardiac ultrasound scan and the target pathology may be a wall motion abnormality in the heart function.

The invention also provides a computer program carrier comprising computer program code which, when executed on a computing device having a processing system, causes the processing system to perform all of the steps of the afore-mentioned method.

The computer program carrier may comprise long-term storage products (e.g., hard drives) or a temporary carrier (e.g. a bitstream).

The invention also provides a system for obtaining an overall score indicative of the presence of a target pathology of a subject during a medical scan procedure, the system comprising a processor configured to:
control a scanning system to acquire images during the medical scan procedure; and
iteratively:
   identify whether a newly acquired image corresponds to one of a set of pre-determined views;
   for a newly acquired image which does correspond to one of the pre-determined views:
      select a predictive model trained on the pre-determined view or predetermined views that have been acquired, from a set of predictive models each trained on one or more of the pre-determined views for the target pathology;
      input the newly acquired image into the selected predictive model; and
      generate or update an overall score indicative of the presence of the target pathology using the output of the selected predictive model.

The processor may be further configured to provide an alert to a clinician performing the medical scan procedure in response to the overall score exceeding a pre-determined threshold and/or the number of acquired images, corresponding to distinct pre-determined views, exceeding a pre-determined view threshold.

The processor may be configured to input the newly acquired image into the selected predictive model to obtain a per-image score indicative of the presence of a target pathology in the newly acquired image and generate or update the overall score using the per-image score, wherein the predictive models are configured to output a per-image confidence measure for the corresponding per-image score and the processor is further configured to iteratively update an overall confidence measure, during the medical scan procedure, with the per-image confidence measures of the acquired images corresponding to one of the pre-determined views.

In response to two or more of the acquired images corresponding to distinct pre-determined views, the processor may be configured to select a predictive model trained on the two or more corresponding pre-determined views from the set of predictive models and input the two or more acquired images into the selected predictive model to obtain a per-image score indicative of the presence of the target pathology in the two or more acquired images.

The medical scan procedure may be a cardiac ultrasound scan and the target pathology may be a wall motion abnormality in the heart function.

The invention also provides a method of assessing the presence of a target pathology of a subject during a medical scan procedure, comprising:
for a newly acquired image of the scan procedure which corresponds to one of a set of pre-determined views:
obtaining an overall score indicative of the presence of the target pathology based on previously acquired images and the newly acquired image, such that the overall score is dynamically and iteratively updated in response to the acquisition of newly acquired images; and
displaying a representation of the overall score together with the newly acquired image.

This method provides a user interface for a clinician which provides an evolving score relating to the presence of a target pathology, so that the clinician has the best available up to date information during a medical scan, on which to base decisions about the scan procedure.

The method may further comprise, for a newly acquired image which corresponds to one of a set of pre-determined views, obtaining an overall confidence measure in respect of the overall score and displaying a representation of the overall confidence measure.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a flowchart for obtaining the overall score indicative of the presence of a target pathology in a subject;
Fig. 2 illustrates an interface to be shown to a clinician;
Figs. 3 and 4 show the results of running predictive models on images of the anterior wall;
Figs. 5 and 6 show the results of running predictive models on images of the lateral wall;
Figs. 7 and 8 show the results of running predictive models on images of the septal wall;
Figs. 9 and 10 show the results of running predictive models on images of the inferior/posterior wall; and
Fig. 11 shows system for obtaining an overall score indicative of the presence of a target pathology.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for obtaining an overall score indicative of the presence of a target pathology of a subject during a medical scan procedure. The method comprises acquiring images during the medical scan procedure and iteratively identifying whether a newly acquired image corresponds to one of a set of pre-determined views. For the newly acquired images which correspond to one of the pre-determined views, a predictive model is selected from a set of predictive models each trained on one or more of the pre-determined views for the target pathology. The selected predictive model is trained on the pre-determined view or multiple predetermined views that have been acquired. The newly acquired image is input into the selected predictive model and an overall score is updated, or generated, using the output of the selected predictive model. The overall score is indicative of the presence of the target pathology in the subject.

By progressively acquiring different views during the exam/medical scan, the sonographer can be informed about the presence of a suspected WMA (or other target pathology). This information is provided by an overall score indicative of the presence of the target pathology. In general, multiple models, trained to operate on a subset of views, are used to iteratively update the overall score. The overall score from the multiple models can be combined with a confidence estimation. This can be derived by a confidence output from the models and/or the completeness of the acquired views. The sonographer can verify that the overall score reached an appropriate confidence before concluding the exam. Additionally, the sonographer can decide whether to stop the exam if the overall score predicts presence of abnormality with acceptable confidence.

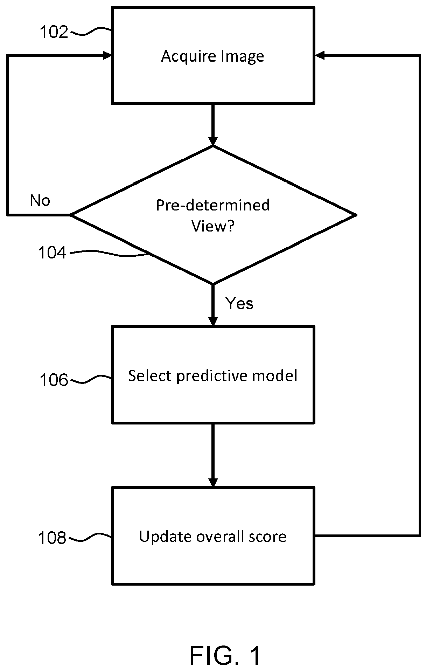

Fig. 1 shows a flowchart for obtaining the overall score indicative of the presence of a target pathology in a subject. In step 102, an image is acquired during a medical scan procedure (herein referred to as a medical scan, a scan or an exam). For various medical scans, there are often various standardized views (i.e. pre-determined views) which can be used by clinicians (e.g. a sonographer, a cardiologist etc.) or predictive models to detect the presence of pathologies.

In step 104, it is determined whether the newly acquired image corresponds to one of the pre-determined views. For example, the image can be input into a view identification model which is trained to identify whether an image corresponds to one of a set of pre-determined views.

The view identification model can be a convolutional neural network (CNN) that is trained in a supervised manner using a labeled dataset. The labeled dataset can contain images that are the input to the view identification model. The known views associated with the images, in the labeled dataset, serve as a ground-truth for training. In an example, the training can be done with a stochastic gradient descent method. Exemplary architectures of the CNN include Visual Geometry Group (VGG)-like, ResNet, DenseNet, EfficientNet and Vision Transformers.

Alternatively, the view identification model could be trained with a self-supervised or unsupervised strategy where no ground-truth annotations are required.

After training, the view identification model takes the newly obtained image as the input and produces a view probability as the output. That probability can be thresholded to obtain a view prediction indicating whether the newly obtained image corresponds to one of the pre-determined views.

If the newly acquired image does not correspond to a pre-determined view, the image can be discarded and the next newly acquired image can be checked.

If the newly acquired image does correspond to a pre-determined view, the image can be further processed. Initially, one or more predictive models are selected in step 106. In particular, predictive models which have been trained on (at least) the pre-determined view of the newly acquired image are selected. For example, consider a set of predictive models where a first predictive model has been trained on a first view (corresponding to the newly acquired image) and a second predictive model has been trained on the first view and a second view. Both of these models could be selected for the newly acquired image as they have both been trained on the first view.

If, later on in time, a second image is acquired (corresponding to the second view), the second model could now be used with only the second image as an input and/or with both the previously acquired first image and the newly acquired second image as inputs.

Similarly to the view identification model, the predictive models could also be CNNs trained in a supervised manner using various labeled datasets or in an unsupervised manner. Here, the labeled datasets may each have images from one or more of the pre-determined views and the ground-truth labels may be diagnoses of the target pathology obtained from clinical experts. As such, each predictive model is trained on one or more of the pre-determined views for the target pathology. Preferably, the set of predictive models contains predictive models trained on only one pre-determined view as well as predictive models trained on more than one pre-determined view.

Thus, the newly acquired image can be input into the selected predictive model(s). Said predictive model(s) will thus output a per-image score for the newly acquired image. The per-image score is indicative of the presence of the target pathology in the newly acquired image.

The per-image score can thus be used to update an overall score in step 108. For example, the overall score may be a combination of all (or a subset) of the per-image scores obtained.

It is noted that the overall score is updated iteratively for the newly acquired images. This takes advantage of the sequential nature of the medical scan (i.e. the images are obtained sequentially during a medical scan). As such, it is not necessary to wait until the end of the medical scan to provide the overall score. This means that the clinician can, for example, stop the medical scan if the overall score indicates the presence of the target pathology. This can limit any damage to the subject caused during the medical scan (e.g. during a stress test for cardiac exams).

As previously discussed, a confidence score can also be provided with the overall score. For example, the predictive models can output a per-image confidence score. The per-image confidence scores can thus be used to update the confidence score. Additionally, the confidence score can be updated based on the number of images at different pre-determined views which have been acquired. The higher the number of images at different pre-determined views, the higher the confidence score may be.

It is thus proposed to present a prediction of abnormalities in the different walls during cardiac echo exams.

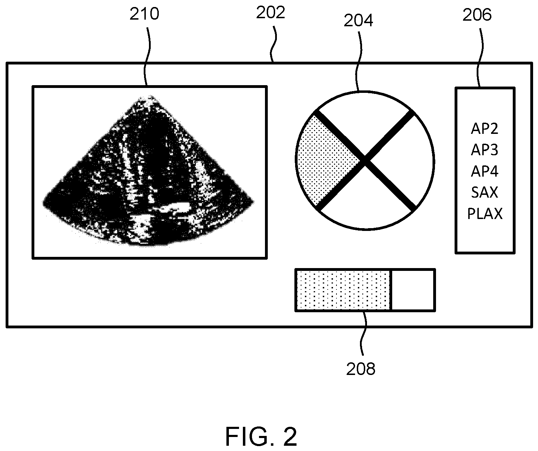

Fig. 2 illustrates an interface 202 shown to the clinician. The predictions 204 here are presented as a circle showing four separate overall scores corresponding to four wall segments (e.g., inferior, lateral, septal and inferior walls). Color coding on the overall scores 204 could be used to depict the level of abnormality. Of course, the predictions 204 could be presented in other ways, such as a list of potential issues found during the exam.

Alternatively, or in addition, a single overall score that combines the scores of the individual walls could be presented. More fine-grained overall scores could also be used. For example, each wall may be separated into the apex, mid and basal region, resulting in three overall scores per wall and, thus, 12 overall scores. In another example, the walls could be divided into 17 overall scores, referred to as the 17 segment model.

A list of expected pre-determined views 206 can also be presented to the sonographer. Of course, a visual encoding can be used to depict the pre-determined views that have been acquired and the ones that remains to be acquired. A quality grading could also be used to present to the sonographer which pre-determined views could be improved upon and which are sufficient.

In this case, the expected pre-determined views include the apical 2 (AP2), apical 3 (AP3), apical 4 (AP4) chamber views as well as the short-axis (SAX) and parasternal long axis (PLAX) views. Of course, other views could also be shown (e.g., the subcostal view or the apical 5 view). In some cases, some views may have sub-categories which can also be shown (e.g. the SAX view can be at basal, mid or apex level and the apical views can be "normal" or "depthreduced").

Finally, a confidence bar 208 can be presented. As mentioned above, the confidence bar 208 may be based on the number of images acquired at different pre-determined views and per-image confidence scores obtained from the predictive models. The confidence bar 208 is meant to increase during the exam. Different visual encodings could also be used.

Newly acquired images 210 can also be shown together with the predictions 204, the list of expected views 206 and the confidence bar 208.

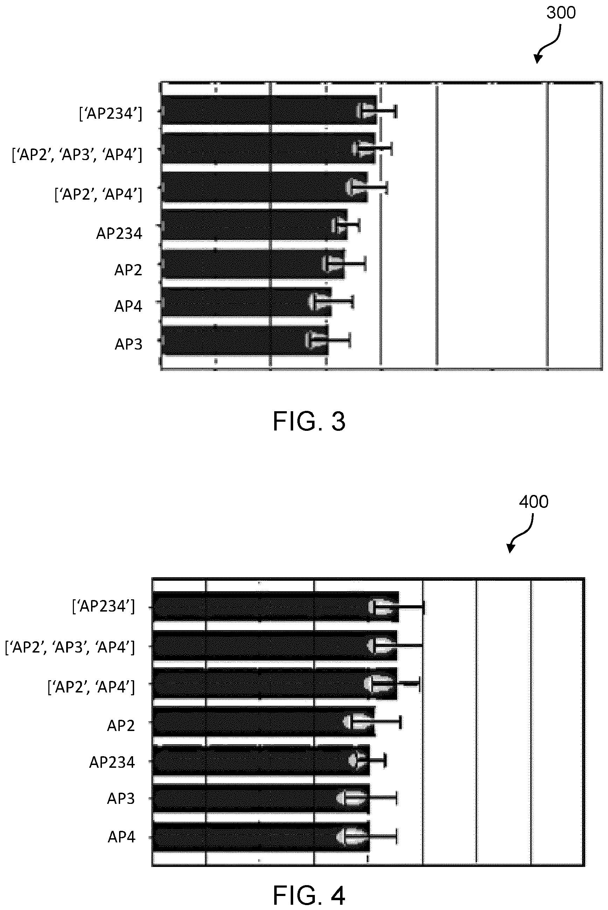

Figs. 3 and 4 show the results 300 and 400 of running predictive models on images of the anterior wall. In particular, the predictive were trained to detect the presence of regional wall motion abnormalities (RWMA) on the anterior wall. Fig. 3 shows the relative area under the curve (AUC) performances 300 for various combinations of predictive models and Fig. 4 shows the relative F1 scores 400 for the same combinations of predictive models. Three different pre-determined views were used - AP2, AP3 and AP4.

The bars corresponding to AP2, AP3 and AP4 show the performances (i.e. the AUC and F1 scores) of per-image scores derived from single views (i.e. views AP2, AP3 and AP4 respectively). The bars corresponding to AP234 show the performances of a predictive model when all views are processed by the same predictive model individually (i.e. evaluated individually).

The bars corresponding to ['AP234'] show the performances of a predictive model when the views AP2, AP3 and AP4 are input into the same predictive model as separate inputs. The outputs of the predictive model can then be averaged to achieve a final output for ['AP234'].

The bars corresponding to ['AP2', 'AP3', 'AP4'] show the performances of three predictive models when the views AP2, AP3 and AP4 are input into different predictive models. The outputs from each of the three predictive models can be averaged to obtain a final output for ['AP2', 'AP3', 'AP4'].

The bars corresponding to ['AP2', 'AP4'] show the performances of two predictive models when the views AP2 and AP4 are input into different predictive models. The outputs from the two predictive models can be averaged to obtain a final output for ['AP2', 'AP4'].

In general, when the notation ["] is used, it means that multiple views are provided as inputs to one or more predictive models and the prediction is combined over such views. Averaging could be used combining the predictions (i.e. the outputs of the predictive models). More advanced ensembling techniques could also be used. For example, the combination of the predictions may include weighted averaging, voting, weighted voting and training a separate machine learning model just for combining the probabilities etc.

It can be observed that using only a single view provides lower performances in terms of AUC and F1. It is worth noting that, although the performance drop is visible, it is not dramatic. When multiple views are used, as in AP234, performance improves. Finally, an increase in performance becomes more substantial when multiple model executions are performed on different views - an approach known as prediction ensembling.

This shows that, by acquiring more information during the exam, predictions become better and more reliable. The confidence score of the prediction can be derived, for example, from entropy measure on the prediction vectors as well as from the number of acquired views and the corresponding model used in the interface.

Therefore, during the exam, the sonographer will start acquiring several views. When such views are automatically recognized by a view identification model, the corresponding RWMA predictive model is selected, and the output put on the interface (as shown in Fig. 2). Then, as more views are acquired and detected, more powerful models can be used, and the confidence can be adjusted accordingly. At the end of the examination, the sonographer will have acquired enough views such that the most powerful model can be used - ['AP234'].

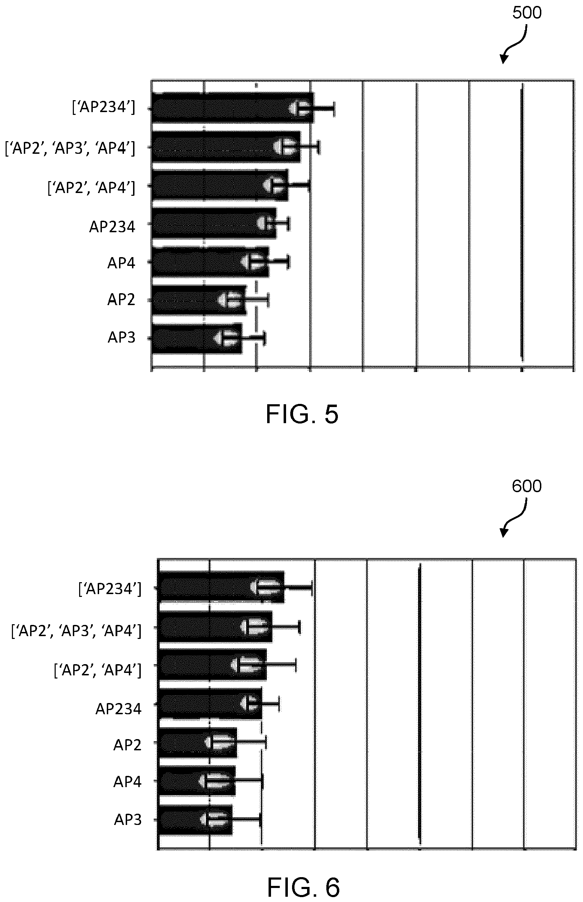

Figs. 5 to 10 show that this concept is also feasible for the lateral, septal and inferior/posterior walls.

Fig. 5 shows the AUC performances 500 for various combinations of predictive models on images of the lateral walls and Fig. 6 shows the corresponding F1 scores 600.

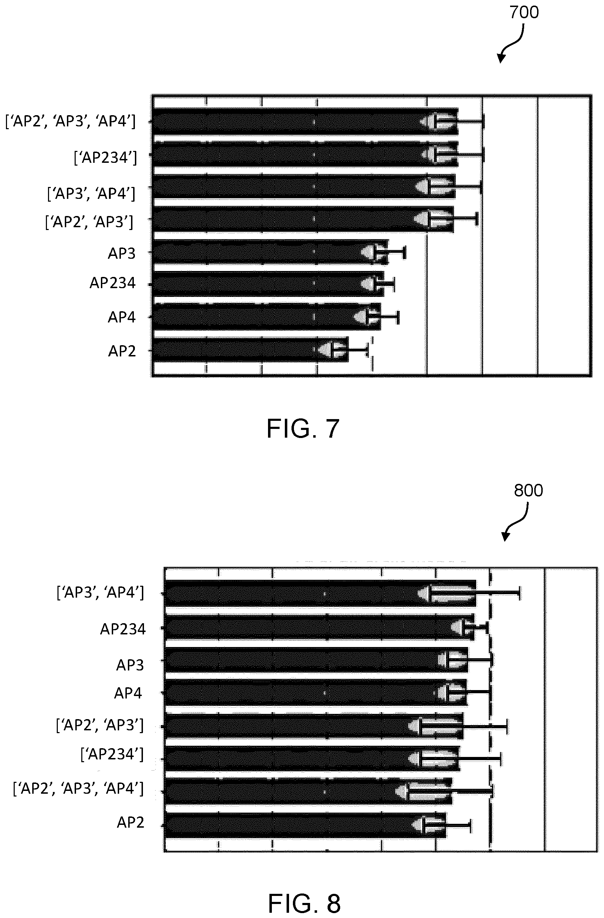

Fig. 7 shows the AUC performances 700 for various combinations of predictive models on images of the septal walls and Fig. 8 shows the corresponding F1 scores 800.

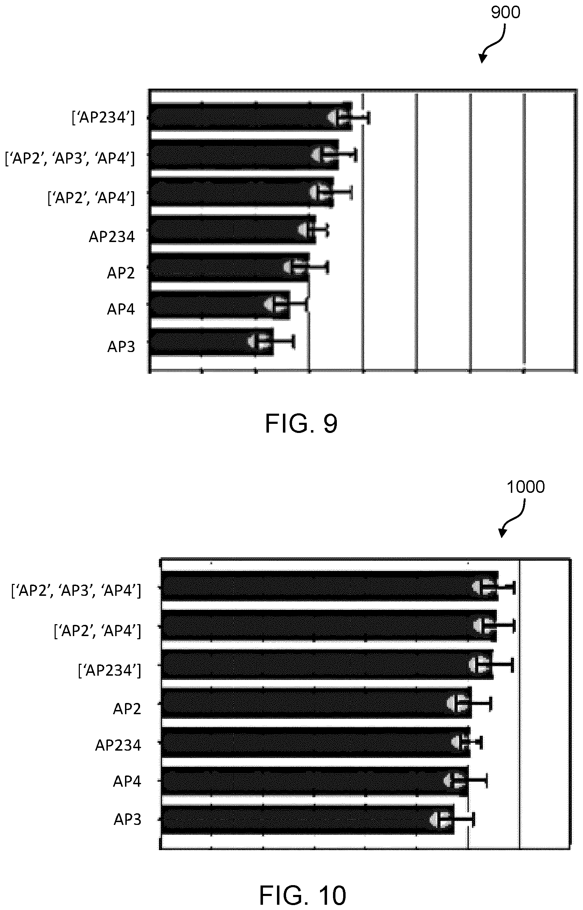

Fig. 9 shows the AUC performances 900 for various combinations of predictive models on images of the inferior/posterior walls and Fig. 10 shows the corresponding F1 scores 1000.

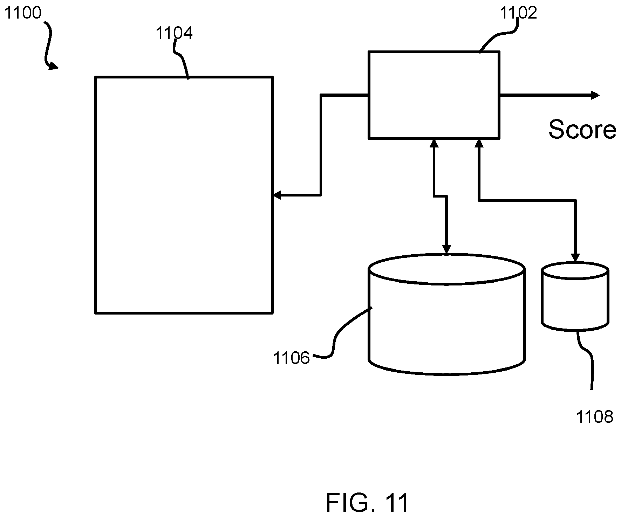

Fig. 11 shows a system 1100 for obtaining an overall score indicative of the presence of a target pathology in a subject during a medical scan procedure. The system comprising a processor 1102 which controls a scanning system 1104 such as an ultrasound scanner to acquire images during the medical scan procedure. The processor 1102 has access to a database 1106 of predictive models and a set 1108 of pre-determined views. When a newly acquired image is obtained from the scanning system, the processor identifies if it corresponds to one of a set of pre-determined views and if so, a most effective predictive model, or set of predictive models, is chosen based on the views that have so far been obtained. The overall score indicative of the presence of the target pathology is then generated (when a first one of the pre-determined views is obtained) or updated.

The previous examples have been provided with respect to detecting RWMA using ultrasound imaging. However, similar progressive acquisitions can be used in the assessment of different pathologies. Additionally, the provided concept can be employed in other imaging modalities providing a live image streaming. Of course, the concept can also be extended to handheld imaging devices, such as point-of-care ultrasound systems.

The processing backend of any herein described method can be placed in the cloud. This is particularly advantageous in the case of a small, handheld, imaging system where the processing resources may be limited by the size of the device (e.g. due to limited cooling on the device). In these cases, the devices could send the newly acquired images to a cloud service. The cloud service could be on premise or in a separate data center. The predictive models can be executed at the cloud service. The cloud service can then send back the overall score(s) to the device for displaying to the user. The newly acquired images could be stored in the cloud service during the medical scan. When new images are received by the cloud service, both the previously received images and the newly acquired images can be used as inputs to one or more predictive models.

The skilled person would be readily capable of developing a processor for carrying out any herein described method. Thus, each step of a flow chart may represent a different action performed by a processor, and may be performed by a respective module of the processor.

As discussed above, the system makes use of processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A method for obtaining an overall score indicative of the presence of a target pathology of a subject during a medical scan procedure, the method comprising:
acquiring (102) images during the medical scan procedure; and
iteratively:
identifying (104) whether a newly acquired image corresponds to one of a set of pre-determined views;
for a newly acquired image which does correspond to one of the pre-determined views:
selecting (106) a predictive model trained on the pre-determined view or multiple predetermined views that have been acquired, from a set of predictive models each trained on one or more of the pre-determined views for the target pathology;
inputting the newly acquired image into the selected predictive model; and
generating or updating (108) an overall score indicative of the presence of the target pathology using the output of the selected predictive model.

2. The method of claim 1, further comprising providing an alert to a clinician performing the medical scan procedure in response to:
the overall score exceeding a pre-determined threshold; or
the number of acquired images, corresponding to distinct pre-determined views, exceeding a pre-determined view threshold.

3. The method of claim 1 or 2, further comprising iteratively updating an overall confidence measure (208), indicating the confidence in the overall score.

4. The method of any one of claims 1 to 3, comprising:
inputting the newly acquired image into the selected predictive model to obtain a per-image score indicative of the presence of a target pathology in the newly acquired image and a per-image confidence measure for the corresponding per-image score; and
generating or updating the overall score and the overall confidence measure using the per-image score and per-image confidence measure.

5. The method of any of claims 3 to 4, wherein iteratively updating the overall score comprises iteratively averaging the latest available per-image score with the overall score.

6. The method of any of claims 1 to 5, comprising, in response to two or more of the acquired images corresponding to distinct pre-determined views:
selecting a predictive model trained on the two or more corresponding pre-determined views from the set of predictive models; and
inputting the two or more acquired images into the selected predictive model to assess the presence of the target pathology in the two or more acquired images.

7. The method of any of claims 1 to 6, wherein the medical scan procedure is a cardiac ultrasound scan and the target pathology is a wall motion abnormality in the heart function.

8. A computer program carrier comprising computer program code which, when executed on a computing device having a processing system, causes the processing system to perform all of the steps of the method according to any of claims 1 to 7.

9. A system for obtaining an overall score indicative of the presence of a target pathology of a subject during a medical scan procedure, the system comprising a processor configured to:
control a scanning system to acquire (102) images during the medical scan procedure; and
iteratively:
identify (104) whether a newly acquired image corresponds to one of a set of pre-determined views;
for a newly acquired image which does correspond to one of the pre-determined views:
select (106) a predictive model trained on the pre-determined view or predetermined views that have been acquired, from a set of predictive models each trained on one or more of the pre-determined views for the target pathology;
input the newly acquired image into the selected predictive model; and
generate or update (108) an overall score indicative of the presence of the target pathology using the output of the selected predictive model.

10. The system of claim 9, wherein the processor is further configured to provide an alert to a clinician performing the medical scan procedure in response to:
the overall score exceeding a pre-determined threshold; or
the number of acquired images, corresponding to distinct pre-determined views, exceeding a pre-determined view threshold.

11. The system of claim 9 or 10, wherein the processor is configured to:
input the newly acquired image into the selected predictive model to obtain a per-image score indicative of the presence of a target pathology in the newly acquired image; and
generate or update the overall score using the per-image score, and
wherein the predictive models are configured to output a per-image confidence measure for the corresponding per-image score and the processor is further configured to iteratively update an overall confidence measure (208), during the medical scan procedure, with the per-image confidence measures of the acquired images corresponding to one of the pre-determined views.

12. The system of any of claims 9 to 11, wherein, in response to two or more of the acquired images corresponding to distinct pre-determined views, the processor is configured to:
select a predictive model trained on the two or more corresponding pre-determined views from the set of predictive models; and
input the two or more acquired images into the selected predictive model to obtain a per-image score indicative of the presence of the target pathology in the two or more acquired images.

13. The system of any of claims 9 to 12, wherein the medical scan procedure is a cardiac ultrasound scan and the target pathology is a wall motion abnormality in the heart function.

14. A method of assessing the presence of a target pathology of a subject during a medical scan procedure, comprising:
for a newly acquired image (210) of the scan procedure which corresponds to one of a set of pre-determined views:
obtaining an overall score (204) indicative of the presence of the target pathology based on previously acquired images and the newly acquired image, such that the overall score is dynamically and iteratively updated in response to the acquisition of newly acquired images; and
displaying a representation of the overall score together with the newly acquired image.

15. The method of claim 14, further comprising:
for a newly acquired image which corresponds to one of a set of pre-determined views:
obtaining an overall confidence measure (208) in respect of the overall score; and
displaying a representation of the overall confidence measure.
